# EUROPEAN PATENT APPLICATION

(11) **EP 1 250 942 A1**
(43) Date of publication of application: **23.10.2002**
(21) Application number: 01201446.0
(22) Date of filing: 19.04.2001
(51) Int. Cl.: A61M 5/50, A61M 5/32

(54) **Safe disposable syringe**

(71) Applicant: Syringe Lab. Co., Ltd., Tortola (VG)
(72) Inventor: Lee, Noner, Peitou Distr., Taipei (TW)
(74) Representative: Prins, Adrianus Willem

(57) **Abstract**

A safe disposable syringe has a hollow barrel (10), a plunger (12), a needle hub (15) with a needle (16) and a safety device. The safety device includes a piston (14) and a stub (122) with a head (124). An oblique face (142) is formed on the piston (14). The stub (122) extends out from the oblique face (142). A hole (154) is defined in the needle hub (15) and aligns with the stub (122). The needle hub (15) will be pulled into the hollow barrel (10) by the engagement of the stub (122) and the hole (154). The needle (16) will incline relative to the longitudinal axis of the barrel (10) due to the oblique face (142). A safety feature is provided. The process for manufacturing the disposable syringe can be simplified. This can decrease the cost for manufacturing the syringe.

## Description

### 1. Field of the Invention

The present invention relates to a disposable syringe, and more particularly to a safe disposable syringe wherein the needle can be pulled into the hollow barrel and inclines relative to the longitudinal axis of the hollow barrel.

### 2. Description of Related Art

A conventional disposable syringe comprises a hollow barrel, a plunger and a needle. The plunger is moveably inserted into one end of the hollow barrel. The needle is mounted on the other end of the barrel and communicates with the barrel. When a user pushes the plunger into the hollow barrel, the medicinal liquid in the barrel can be ejected from the needle by the pressure provided by the plunger. In addition, a cap is always mounted on the hollow barrel to cover the needle, such that the used needle will not puncture people.

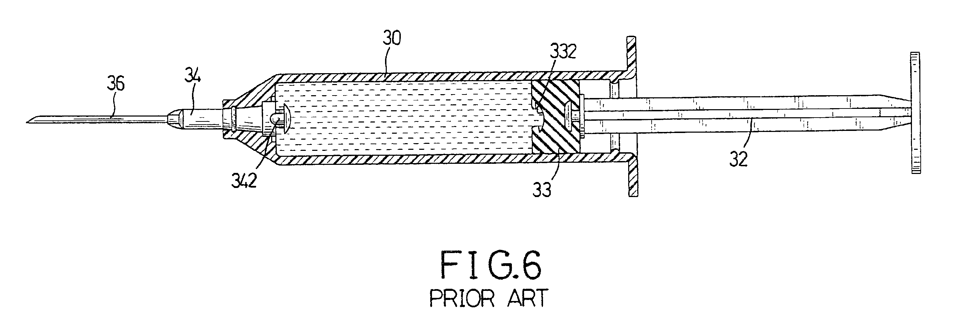

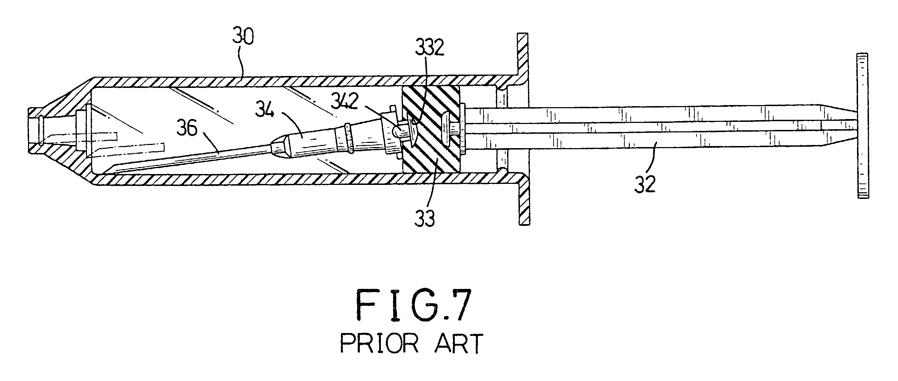

However, the user is still easily punctured by the needle when he or she puts the cap onto the hollow barrel. Therefore, a safety device is provided with the conventional disposable syringe to prevent the user from being punctured. With reference to Figs. 6 and 7, the disposable syringe with a conventional safety device comprises a hollow barrel (30), a plunger (32), a needle hub (34) and a needle (36). The plunger (32) is moveably inserted into one end of the hollow barrel (30). A piston (33) is secured to the end of the plunger (32) that extends into the open end of the barrel (30). The needle hub (34) is detachably mounted on the other end of the barrel (30). The needle (36) is secured to the needle hub (34). An inclined recess (332) is defined in the piston (33). A resilient button hook (342) is formed on the needle hub (34) and aligns with the recess (332) in the piston (33). When the plunger (32) is fully pushed into the hollow barrel (30), the button hook (342) will deform and engage with the recess (332). The needle hub (34) with the needle (36) will release from and be pulled fully into the barrel (30) when the plunger (32) is pulled back. The used needle (36) will be fully enclosed and held in the hollow barrel (30) to keep the needle (36) from touching or puncturing anyone. Because the recess (332) is inclined, the needle (36) will not be aligned with the hole in the hollow barrel (30) after it is pulled into the barrel (30). When the user subsequently pushes the plunger (32) into the hollow barrel (30) again, the needle (36) will be bent and the size of the used syringe is reduced.

Even though the conventional disposable syringe with a safety device can keep the used needle (36) from inadvertently puncturing anyone, the recess (332) in the piston (33) must be formed with a complex shape. The mold for manufacturing the piston (33) with the inclined recess (332) is complex, and the process of forming the plunger (32) is difficult. The cost for manufacturing the conventional disposable syringe with the safety device will increase.

To overcome the shortcomings, the present invention tends to provide an improved safe disposable syringe to mitigate or obviate the aforementioned problems.

The main objective of the invention is to provide a disposable syringe with an improved safety device to simplify the process for manufacturing the syringe. The syringe has a hollow barrel, a plunger, needle hub and a safety device. The plunger is moveably inserted into one end of the hollow barrel. The needle hub is detachably mounted on the other end of the barrel. The safety device includes a piston, a stub with a head and a hole defined in the needle hub. An oblique face is formed on the end of the piston. The stub extends out from the oblique face of the piston. The hole is defined in the needle hub and aligns with the stub. The needle hub with the needle will be pulled into the hollow barrel when the stub engages with the hole, and the needle will incline relative to the longitudinal axis of the barrel due to the oblique face on the piston. This not only provides a safety feature to the disposable syringe, but also simplifies the process for manufacturing the disposable syringe.

Other objectives, advantages and novel features of the invention will become more apparent from the following detailed description when taken in conjunction with the accompanying drawings.

### IN THE DRAWINGS:

Fig. 1 is an exploded perspective view of a safe disposable syringe in accordance with the present invention;
Fig. 2 is an enlarged cross sectional partial side plan view of the syringe in Fig. 1;
Fig. 3 is a side plan view in partial section of the syringe in Fig. 1 showing the stub engaged with the recess defined in the plug;
Fig. 4 is a side plan view in partial section of the syringe in Fig. 1 showing the plug, needle hub and needle in the hollow barrel and the needle inclining relative to the longitudinal axis of the barrel;
Fig. 5 is a side plan view in partial section of the syringe in Fig. 1 showing that the needle is bent when the plunger is pushed into the hollow barrel;
Fig. 6 is a side plan view in partial section of a conventional safe disposable syringe in accordance with the prior art; and
Fig. 7 is an operational side plan view in partial section of the convention syringe in Fig. 6.

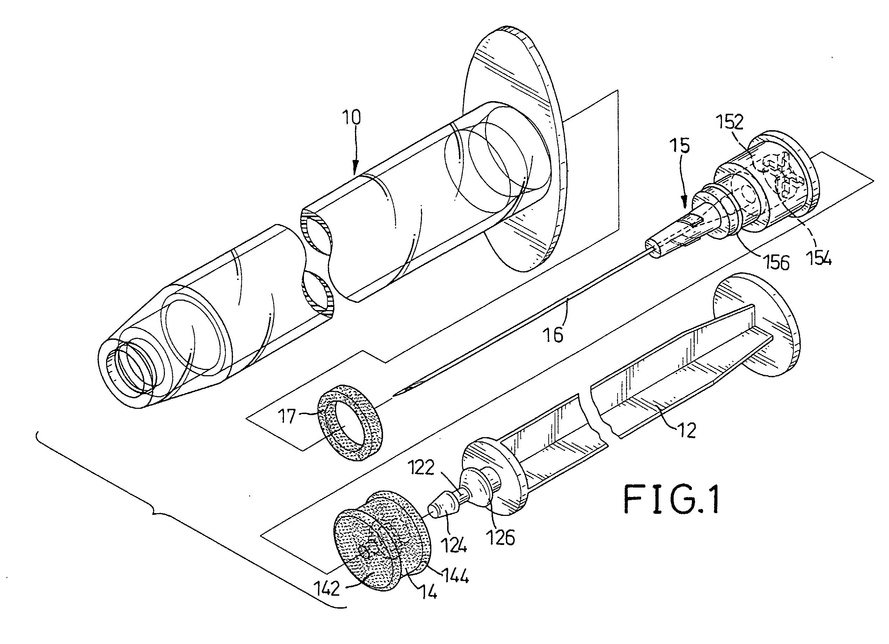

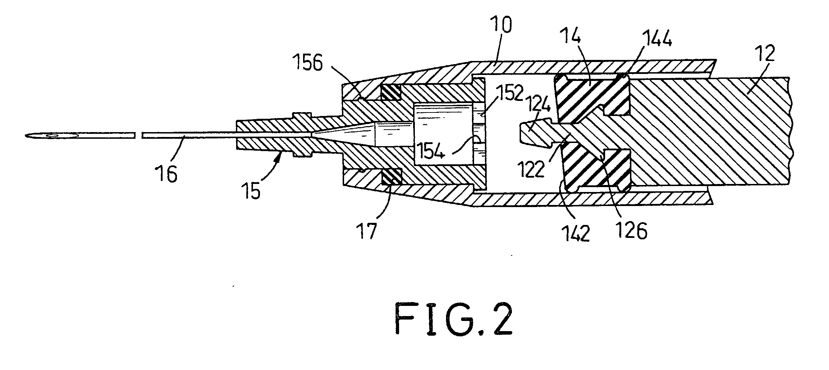

With reference to Figs. 1 and 2, a safe disposable syringe in accordance with the present invention comprises a hollow barrel (10), a plunger (12), a needle hub (15) and a needle (16). The plunger (12) is moveably inserted into one end of the hollow barrel (10), and the needle hub (15) is detachably mounted on the other end of the barrel (10).

A separate piston (14) made of a flexible material is attached to the end of the plunger (12) that extends into the barrel (10). An oblique face (142) is defined on the free side of the piston (14). A through hole (not numbered) is defined through the piston (14). A stub (122) with an enlarged head (124) extends longitudinally from the end of the plunger (12) that extends into the barrel (10). The stub (122) extends through the through hole in the piston (14) and out from the oblique face (142) of the piston (14). In practice, a flange (126) is formed on the middle portion of the stub (122). A recess (not numbered) is defined in the inner wall of the through hole of the piston (14) to receive the flange (126) on the stub (122). Consequently, the piston (14) will be securely attached to the stub (122) and will not release from the stub (122) unintentionally. At least one O-ring (144) is integrally formed around the outer periphery of the piston (14) to seal the gap defined between the hollow barrel (10) and the piston (14).

The needle hub (15) is a hollow body and communicates with the inside of the hollow barrel (10). A cross bar (152) is formed on the end of the needle hub (15) extending into the barrel (10). A hole (154) is defined in the bar (152) and aligns with the stub (122). A flexible gasket (17) is pressed onto the needle hub (15) to seal the gap between the inner wall of the barrel (10) and the needle hub (15). A rib (156) is formed around the outer periphery of the needle hub to engage with the inner wall of the hollow barrel (10). The combination between the needle hub (15) and the barrel (10) is enhanced.

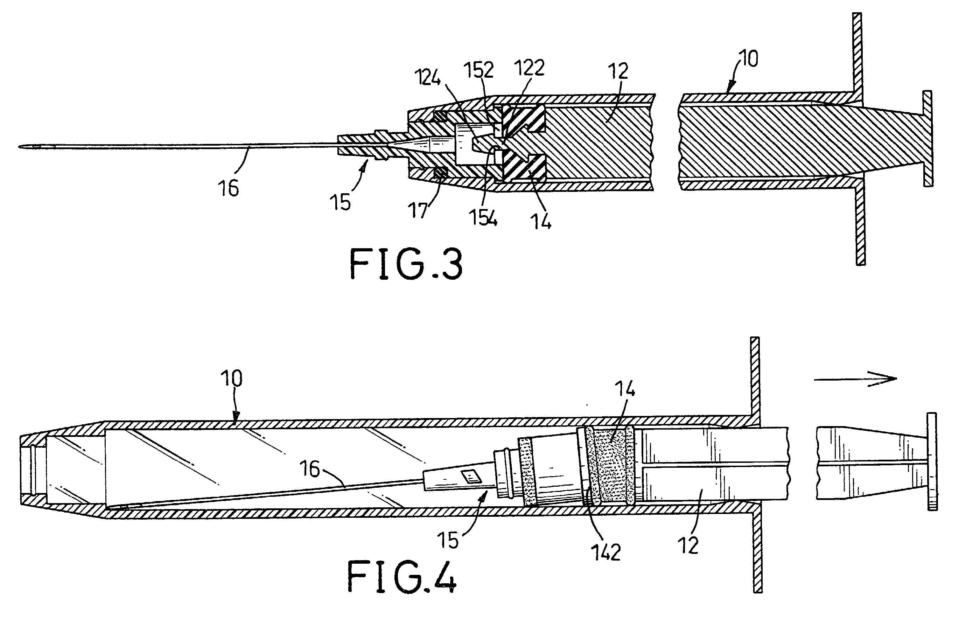

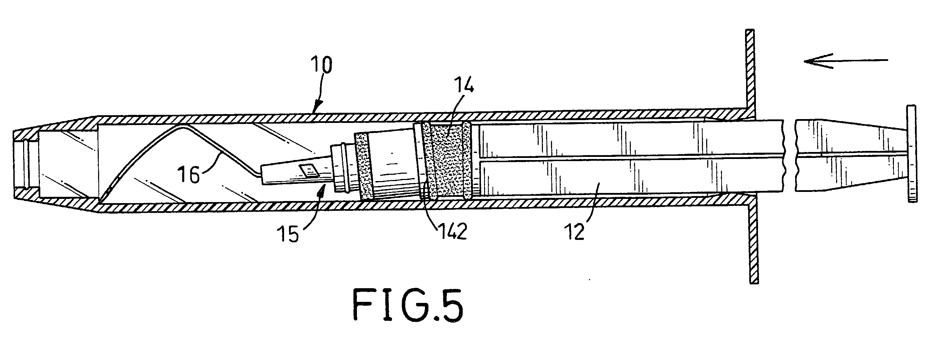

With reference to Figs. 1 to 4, when the plunger (12) is fully pushed into the hollow barrel (10), the head (124) of the stub (122) will engage with the hole (154) in the needle hub (15). When the plunger (12) is pulled back, the needle hub (15) with the needle (16) will be released from the end of the hollow barrel (10) and received in the hollow barrel (10). By such an arrangement, the used needle (16) is not exposed, and no one will touch or be punctured by the needle (16). This protects the person who disposes of the used syringe from being infected with disease by the used needle (16).

With reference to Figs. 2, 4 and 5, because an oblique face (142) is defined on the piston (14) and the piston (14) is made of a flexible material, the oblique face (142) of the piston (14) will deform due to the abutment of the needle hub (15) as the head (125) of the stub (124) engages with the hole (154). When the needle hub (15) is pulled into the barrel (10), the needle hub (15) with the needle (16) will incline relative to the hollow barrel (10) due to the return of the oblique face (142) to its original incline. The needle (16) will be bent when the plunger (12) is pushed into the barrel (10) again. The length of the used disposable syringe is reduced. In addition, the mold to form a plunger (12) with a stub (122) and a piston (14) with an oblique face (122) are simpler than forming a plunger (32) with a piston (33) with an inclined recess (332) as shown in Figs. 6 and 7. The cost for manufacturing the safe disposable syringe can be reduced.

Even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A safe disposable syringe comprising:
a hollow barrel having a first end and a second end;
a plunger with two ends moveably inserted into the first end of the hollow barrel;
a stub with an enlarged head formed on the plunger;
a needle hub detachably mounted on the second end of the barrel to communicate with the hollow barrel and having a hole defined in the needle hub at an end extending into the barrel to be adapted to engage with the stub as the plunger is fully pushed into the hollow barrel;
a needle secured to the needle hub; and
a piston deformably and detachably mounted on the plunger at the end extending into the barrel and having an oblique face formed in a manner that when the hub is pulled backward into the barrel, the hub will be tilted by the oblique of the piston,
whereby the needle hub and the needle can be pulled into the hollow barrel to provide a safety effect.

2. The safe disposable syringe as claimed in claim 1, wherein a gasket is pressed on the needle hub to seal a gap defined between the hollow barrel and the needle hub.

3. The safe disposable syringe as claimed in claim 1, wherein a through hole is defined through the piston for the stub to extend through the through hole.

4. The safe disposable syringe as claimed in claim 3, wherein at least one O-ring is arranged around an outer periphery of the piston to seal a gap defined between the barrel and the piston.

5. The safe disposable syringe as claimed in claim 3, wherein a flange is formed on a middle portion of the stub; and
a recess is defined in an inner wall of the through hole of the piston to receive the flange on the stub so as to securely attach the piston to the stub.

6. The safe disposable syringe as claimed in claim 1, wherein a cross bar is formed on the end of the needle hub extending into the barrel; and
the hole facing the stub is defined in the bar.
